# EUROPEAN PATENT SPECIFICATION

(11) **EP 3 766 442 B1**
(45) Date of publication and mention of the grant of the patent: **05.06.2024**
(21) Application number: 18909838.7
(22) Date of filing: 14.03.2018
(51) Int. Cl.: A61B 17/3207, A61B 17/22

(54) **SHAFT FOR IN VIVO RECOVERY MECHANISM**
SCHAFT FÜR EINEN IN-VIVO-WIEDERHERSTELLUNGSMECHANISMUS
ARBRE POUR MÉCANISME DE RÉCUPÉRATION IN VIVO

(43) Date of publication of application: 20.01.2021
(73) Proprietor: ASAHI INTECC CO., LTD., Seto-shi, Aichi 489-0071 (JP)
(72) Inventor: NISHIHARA, Hiroyuki, Seto-shi, Aichi 489-0071 (JP); HANAOKA, Atsuhiro, Seto-shi, Aichi 489-0071 (JP)
(74) Representative: Winter, Brandl - Partnerschaft mbB
(86) International application number: PCT/JP2018/009840
(87) International publication number: WO 2019/175996

(56) References cited:
- WO-A1-2010/115134
- WO-A2-2008/005888
- JP-A- 2014 233 312
- JP-A- 2017 521 153
- US-A- 5 902 263
- US-A1- 2012 108 902
- US-A1- 2014 148 830
- US-A1- 2014 188 127

## Description

### TECHNICAL FIELD

The present invention relates to a shaft for an in vivo recovery mechanism for use in an in vivo recovery mechanism that removes a substance from a body lumen.

### BACKGROUND ART

Conventionally, various devices have been developed that remove a substance from a body lumen of a patient. For example, in Patent Document 1, a device for removing a substance from a body lumen is described which includes a catheter having a proximal end, a distal end, and a catheter lumen that extends therethrough, a cutter assembly which is rotatably joined to the distal end of the catheter, and a rotatable torque shaft having a first end, which extends through the catheter lumen and is joined to the cutter assembly, and a second end which is configured so as to be joined to a rotation mechanism, wherein an outer coil is provided on an outer surface of the torque shaft, and the outer coil is spirally formed on the outer surface of the torque shaft such that a substance inside a body lumen is transported in the proximal direction when the torque shaft is rotated (see FIG. 20, etc.). Patent Document 2 relates to an apparatus and methods for removing occluding material from stented regions within blood vessels, which have restenosed. Patent Document 3 discloses a medical device including a tubular braided fashion sleeve where a plurality of wires such as 2, 3, 4 or 5 can be stranded together. Patent Document 4 describes a shaft for draining genitourinary organs. Patent Document 5 discloses devices and methods for removal of the occluding material from the blood vessels as well as other body lumens. Patent Document 6 discloses systems and methods for minimally invasive discectomy procedures. Patent Document 7 relates to an atherectomy device capable of opening an opening in a chronic total occlusion.

### CITATION LIST

### Patent Document

Patent Document 1: Japanese Unexamined Patent Application Publication No. 2013-138877
Patent Document 2: US 5 902 263 A1
Patent Document 3: US 2014/0188127 A1
Patent Document 4: US 2012/0108902 A1
Patent Document 5: WO 2008/005888 A2
Patent Document 6: WO 2010/115134 A1
Patent Document 7: JP 2017 521 153 A

### SUMMARY OF INVENTION

### Problems to be Solved by the Invention

The device described in Patent Document 1 (hereinafter referred to as "in vivo recovery mechanism") discharges a substance inside a body lumen (hereinafter referred to as "substance in the body") to the outside of a patient's body by means of the outer coil spirally formed on the torque shaft, and the performance for recovering and transporting the substance in the body depends on the outer coil.

Furthermore, in procedures and surgeries, it is vital to quickly perform the work of recovering and transporting the substance in the body, and enhancements that enable the recovery and transport performance to be further improved are desired.

The present invention has been made in response to the above problems associated with the conventional technique, and an object of the present invention is to provide a shaft for an in vivo recovery mechanism that improves the recovery and transport performance of an in vivo recovery mechanism to enable in vivo recovery work in procedures and surgeries to be quickly and reliably performed.

### Means for Solving the Problem

This object is solved by the subject matter of the independent claim. Further aspects are disclosed in the dependent claims.

In order to solve the above problems, a shaft for an in vivo recovery mechanism for removing a substance from a body lumen according to the invention (referred to also as first aspect of the invention) includes a shaft, and a side wire sparsely wound onto an outer circumference of the shaft, and having protruding and recessed parts on an outer circumferential surface of the side wire, wherein the side wire is constitued by a first strand formed by twisting a plurality of first wires, and the protruding and recessed parts of the side wire are formed from an outline of the first strand formed by twisting a plurality of first wires, the shaft is constituted by a second strand formed by twisting a plurality of second wires, or the shaft is constituted by a hollow strand formed by twisting a plurality of third wires.

Moreover, a second aspect of the present invention is the shaft for an in vivo recovery mechanism according to the first aspect, wherein a twisting direction of the first strand is the same direction as a winding direction of the side wire onto the shaft.

Also, a third aspect of the present invention is the shaft for an in vivo recovery mechanism according to the first aspect or the second aspect, wherein protruding and recessed parts are formed on an outer circumferential surface of the wires constituting the first strand.

In addition, a fourth aspect of the present invention is the shaft for an in vivo recovery mechanism according to any one of the first aspect to the third aspect, wherein the protruding and recessed parts have protruding parts and recessed parts, which extend along a direction substantially perpendicular to a long axis direction of the shaft, in a direction parallel to the long axis direction.

### Effects of the Invention

The shaft for an in vivo recovery mechanism according to the first aspect of the present invention is provided with a shaft, and a side wire sparsely wound onto the outer circumference of the shaft and having protruding and recessed parts on the outer circumferential surface thereof, and therefore, when rotating the shaft for an in vivo recovery mechanism, a substance in the body is caught between the sparsely wound side wire on the shaft and held by the protruding and recessed parts formed on the side wire, and the performance for recovering and transporting the substance in the body can be improved.

Furthermore, the side wire is constituted by a first strand formed by twisting a plurality of first wires, and the protruding and recessed parts of the side wire are formed from an outline of the first strand, and therefore, the protruding and recessed parts of the side wire are easily formed, and when rotating the shaft for an in vivo recovery mechanism, a substance in the body is caught between the sparsely wound side wire on the shaft and held by the protruding and recessed parts formed from the outline of the first strand, and the performance for recovering and transporting the substance in the body can be improved.

Moreover, the second aspect of the present invention is the shaft for an in vivo recovery mechanism according to the first aspect, wherein a twisting direction of the first strand is the same direction as a winding direction of the side wire onto the shaft, and therefore, the protruding and recessed parts have protruding parts and recessed parts, which are perpendicularly inclined in a direction parallel to the long axis of the shaft, and when rotating the shaft for an in vivo recovery mechanism, in addition to the effects of the shaft for an in vivo recovery mechanism of the first aspect, the performance for recovering and transporting the substance in the body can be further improved.

Also, the third aspect of the present invention is the shaft for an in vivo recovery mechanism according to the first aspect or the second aspect, wherein protruding and recessed parts are formed on an outer circumferential surface of the wires constituting the first strand, and therefore, in addition to the effects of the shaft for an in vivo recovery mechanism of the first aspect or the second aspect, the performance for recovering and transporting the substance in the body can be further improved.

In addition, the fourth aspect of the present invention is the shaft for an in vivo recovery mechanism according to any one of the first aspect to the third aspect, wherein the protruding and recessed parts have protruding parts and recessed parts, which extend along a direction substantially perpendicular to a long axis direction of a hollow shaft, in a direction parallel to the long axis direction, and therefore, when rotating the shaft for an in vivo recovery mechanism, in addition to the effects of the shaft for an in vivo recovery mechanism of any one of the first aspect to the third aspect, the performance for recovering and transporting the substance in the body can be further improved.

Furthermore, the shaft for an in vivo recovery mechanism of the present invention is constituted by a second strand formed by twisting a plurality of second wires, and therefore, the shaft for an in vivo recovery mechanism is made softer, and further, when rotating the shaft for an in vivo recovery mechanism, the substance in the body is caught between the sparsely wound side wire on the shaft and held by the protruding and recessed parts formed from the second strand of the shaft and the protruding and recessed parts formed on the side wire, and the performance for recovering and transporting the substance in the body can be further improved.

Alternatively, the shaft for an in vivo recovery mechanism of the present invention is constituted by a hollow strand formed by twisting a plurality of third wires, and therefore, the shaft for an in vivo recovery mechanism is made even softer, and further, when rotating the shaft for an in vivo recovery mechanism, the substance in the body is caught between the sparsely wound side wire on the shaft and held by the protruding and recessed parts formed from the hollow strand of the shaft and the protruding and recessed parts formed on the side wire, and the performance for recovering and transporting the substance in the body can be further improved. Furthermore, by inserting a guide wire or the like into a void inside the hollow strand and causing the hollow strand to be positioned along the guide wire, the shaft for an in vivo recovery mechanism is capable of reaching the periphery of a body lumen such as a blood vessel.

### BRIEF DESCRIPTION OF DRAWINGS

FIG. 1 is an external view of an in vivo recovery mechanism using a shaft for an in vivo recovery mechanism according to a first embodiment of the present invention.
FIG. 2 is an explanatory diagram of the inside of section A in FIG. 1.
FIG. 3 is an enlarged view of the shaft for an in vivo recovery mechanism of the first embodiment.
FIG. 4 is an enlarged view of a shaft for an in vivo recovery mechanism according to a first embodiment.
FIG. 5 is an enlarged view of a shaft for an in vivo recovery mechanism according to a second embodiment.
FIG. 6 is an enlarged view of a shaft for an in vivo recovery mechanism according to a third embodiment.
FIG. 7 is an enlarged view of a shaft for an in vivo recovery mechanism according to a fourth embodiment.
FIG. 8 is an enlarged view of a shaft for an in vivo recovery mechanism according to a first embodiment.
FIG. 9 is an enlarged view of a shaft for an in vivo recovery mechanism according to a first embodiment.

### EMBODIMENTS OF THE INVENTION

### Best Mode for Carrying Out the Invention

Hereinafter, embodiments of the present invention will be described with reference to the drawings.

### (First Embodiment)

First, a first embodiment of the present invention will be described.

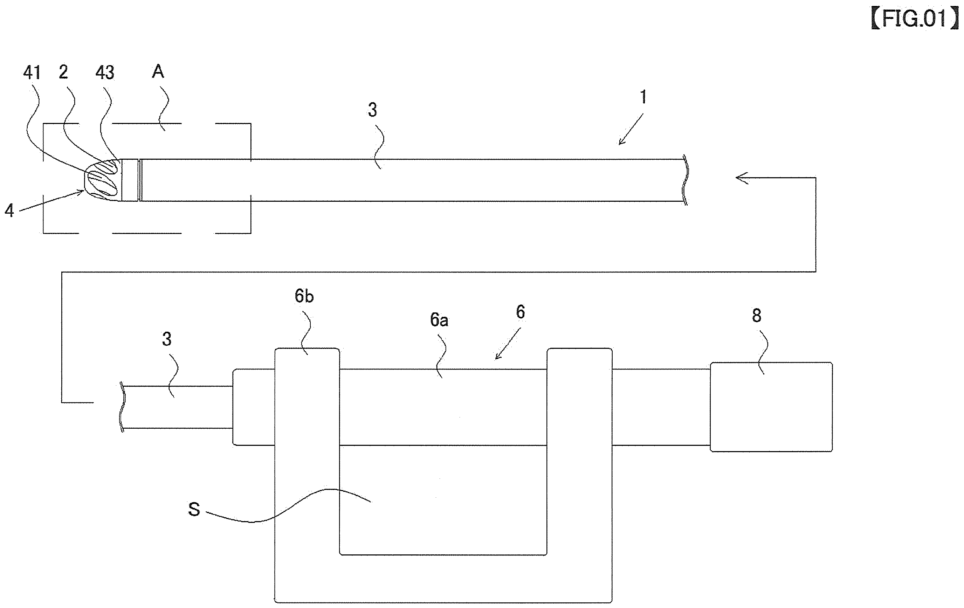

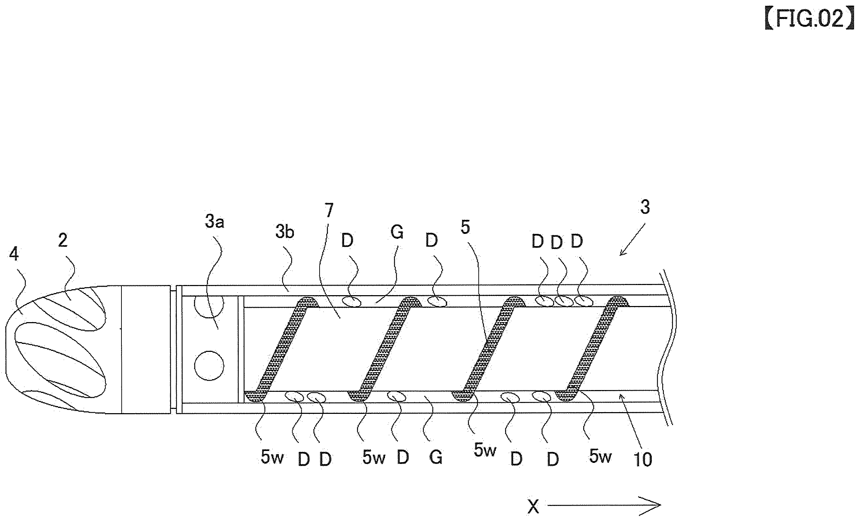

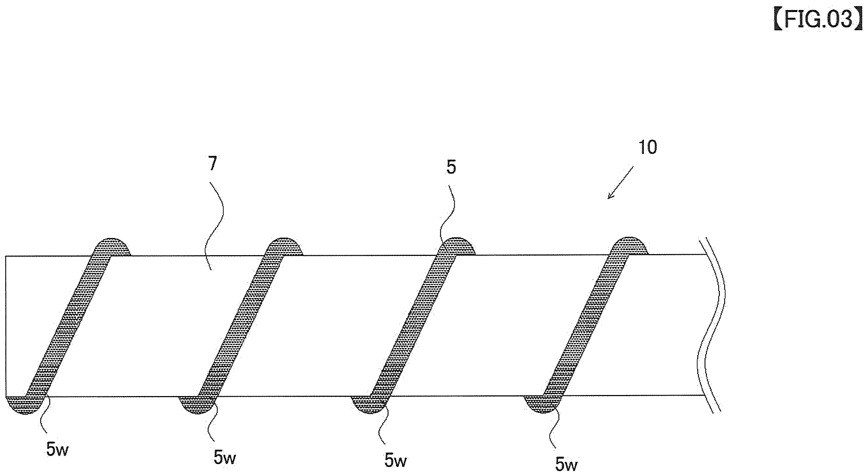

FIG. 1 is an external view of an in vivo recovery mechanism using a shaft for an in vivo recovery mechanism according to the first embodiment of the present invention, FIG. 2 is an explanatory diagram of the inside of section A in FIG. 1, and FIG. 3 is an enlarged view of the shaft for an in vivo recovery mechanism according to the first embodiment.

As shown in FIG. 1, the in vivo recovery mechanism 1 of the present invention for removing a substance from a body lumen includes a catheter 3 having a long hollow tubular body, a cutter assembly 4 connected to the distal end of the catheter 3, a grip part 6 connected to the proximal end of the catheter 3, and a motor 8 connected to the proximal end of the grip part 6.

The cutter assembly 4 includes a plurality of openings 41, a casing 43 connected to the distal end of the catheter 3, and a cutter 2 disposed inside the casing 43. The cutter 2 is connected to the distal end of the shaft 10 for an in vivo recovery mechanism described below (see FIG. 2), and is capable of rotating with the rotation of the motor 8.

Therefore, the cutter assembly 4 cuts a substance in the body D (see FIG. 2), such as plaque, that has entered from the openings 41 of the casing 43 by means of the rotating cutter 2, and then takes the substance into the interior of the cutter assembly 4.

The grip part 6 is connected to the proximal end of the catheter 3, and includes a grip part main body 6a, and a grip part side body 6b, which is letter U-shaped and is connected to the grip part main body 6a. Furthermore, a gap S, which is can be gripped by the person performing the procedure, is formed in the grip part 6 by the grip part main body 6a and the grip part side body 6b.

Moreover, the casing of the motor 8 is connected to the proximal end of the grip part 6, and the rotation shaft of the motor 8 is connected to the proximal end of the shaft 10 for an in vivo recovery mechanism described below (see FIG. 2).

In FIG. 1, the cutter assembly 4 is linearly connected to the catheter 3, and although the mechanism is not shown, in reality the cutter assembly 4 is capable of being bent by an operation of the grip part 6 in all 360 degrees with respect to the long axis of the catheter 3.

The catheter 3 includes a catheter main body 3b, a bearing 3a connected to the interior of the distal end of the catheter main body 3b, and a bearing (not shown) connected to the interior of the proximal end of the catheter main body 3b. Furthermore, the catheter 3 has the distal end connected to the cutter assembly 4, the proximal end connected to the grip part 6, and the interior is provided with the rotatable shaft 10 for an in vivo recovery mechanism.

As shown in FIG. 2, the shaft 10 for an in vivo recovery mechanism is constituted by a shaft 7 and a side wire 5 sparsely wound onto the outer circumference of the shaft 7, and is capable of rotating inside the catheter 3 with the rotation of the motor 8.

The shaft 7 is formed from a single long metallic wire, and is connected to the proximal end of the cutter 2 through the bearing 3a, which is connected to the interior of the distal end of the catheter 3. Although the material of the shaft 7 is not particularly limited if it is a biocompatible material such as stainless steel, Ni-Ti alloy, or cobalt alloy, stainless steel is used in the present embodiment.

The shaft 7 may be formed from a long solid metal wire, or may be formed from a long hollow metal wire. However, the shaft 10 for an in vivo recovery mechanism can be made softer by forming the shaft 7 with a long hollow metallic wire, and by inserting a guide wire or the like into a void inside the hollow shaft and causing the hollow shaft to be positioned along the guide wire, the shaft 10 for an in vivo recovery mechanism is capable of reaching the periphery of a body lumen such as a blood vessel.

Fine protruding and recessed parts 5w are formed on the outer circumferential surface of the side wire 5, and the area in which the protruding and recessed parts 5w are formed is indicated by a hatching pattern in FIG. 2 and FIG. 3. That is to say, the protruding and recessed parts 5w in the present embodiment are formed on the entire outer circumferential surface of the side wire 5.

In the present embodiment, although the protruding and recessed parts 5w are formed on the entire outer circumferential surface of the side wire 5, they may be formed on part of the outer circumferential surface of the side wire 5. However, when the protruding and recessed parts 5w are formed on the entire outer circumferential surface of the side wire 5, it is possible to improve the retention performance of the substance in the body and improve the recovery and transport performance to a greater extent.

Furthermore, although the material of the side wire 5 is not particularly limited if it is a biocompatible material such as stainless steel, tungsten, or Ni-Ti alloy, stainless steel is used in the present embodiment.

Moreover, because a gap G is formed between the catheter 3 and the shaft 10 for an in vivo recovery mechanism, the substance in the body D, such as plaque, which is cut by the cutter 2 and taken into the interior of the cutter assembly 4, is retained inside the gap G, and is transported in the X direction by the rotation of the shaft 10 for an in vivo recovery mechanism.

The shaft 10 for an in vivo recovery mechanism is provided with the shaft 7, and the side wire 5 sparsely wound onto the outer circumference of the shaft 7 and having protruding and recessed parts 5w on the outer circumferential surface thereof, and therefore, when rotating the shaft 10 for an in vivo recovery mechanism, the substance in the body D is caught between the sparsely wound side wire 5 on the shaft 7 and held by the protruding and recessed parts 5w formed on the side wire 5, and the performance for recovering and transporting the substance in the body can be improved.

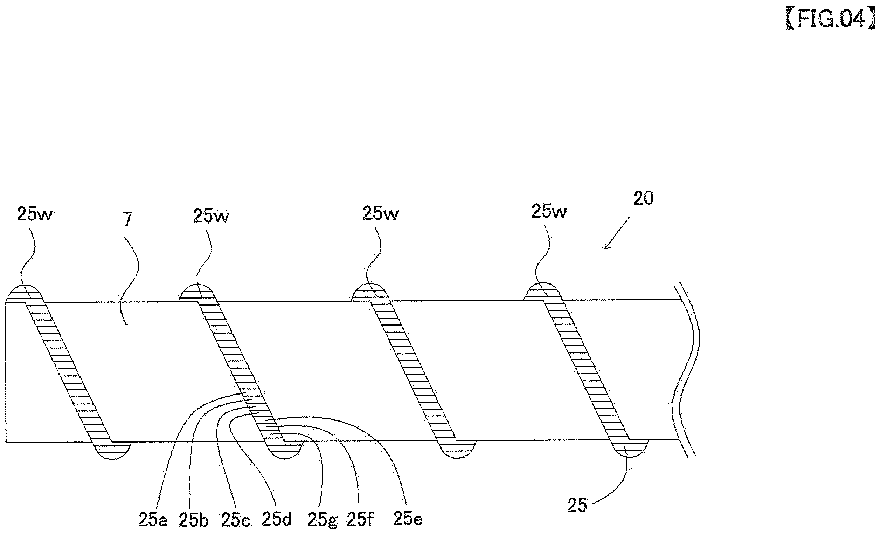

FIG. 4 is an enlarged view of a shaft for an in vivo recovery mechanism according to the first embodiment.

Hereinafter, the first embodiment of the present invention will be described, however, the in vivo recovery mechanism used in the shaft for an in vivo recovery mechanism of the present embodiment is identical to that of the shaft for an in vivo recovery mechanism 1 of the first embodiment, and the interior of the distal end of the in vivo recovery mechanism 1 is identical to FIG. 2, and therefore, the description thereof will be omitted, and further, those parts that are the same as the first embodiment will be referred to by the same reference numerals, and the description will be omitted.

The shaft 20 for an in vivo recovery mechanism of the present embodiment has the distal end connected to the cutter 2 through the bearing 3a connected to the interior of the distal end of the catheter main body 3b, the proximal end connected to the motor 8 through a bearing (not shown) connected to the interior of the proximal end of the catheter main body 3b, and is capable of rotating with the rotation of the motor 8.

As shown in FIG. 4, the shaft 20 for an in vivo recovery mechanism is constituted by the shaft 7 and a strand 25 (corresponding to a "first strand" of the present invention) serving as the side wire sparsely wound onto the outer circumference of the shaft 7, and the strand 25 is formed by twisting seven wires (corresponding to "first wires" of the present invention) 25a, 25b, 25c, 25d, 25e, 25f, and 25g.

Furthermore, the twisting direction of the strand 25 is a counterclockwise direction (hereinafter, referred to as "S-twisting") toward the left direction in the drawing, and the winding direction of the strand 25 onto the shaft 7 is a clockwise winding (hereinafter, referred to as "Z-winding") toward the left direction of the drawing, such that the twisting direction of the strand 25 and the winding direction of the strand 25 onto the shaft 7 are opposite directions.

Protruding and recessed parts 25w are formed from the outline of the strand 25, and the protruding and recessed parts 25w are formed around the entire outer circumferential surface of the strand 25.

Furthermore, although the material of the wires (corresponding to "first wires" of the present invention) 25a, 25b, 25c, 25d, 25e, 25f, and 25g constituting the strand 25 is not particularly limited if it is a biocompatible material such as stainless steel, tungsten, or Ni-Ti alloy, stainless steel is used in the present embodiment.

The shaft 20 for an in vivo recovery mechanism is provided with the shaft 7, and the strand 25 sparsely wound onto the outer circumference of the shaft 7 and having protruding and recessed parts 25w on the outer circumferential surface thereof, and therefore, the protruding and recessed parts 25w of the strand 25 can be easily formed, and when rotating the shaft 20 for an in vivo recovery mechanism, the substance in the body D is caught between the sparsely wound strand 25 on the shaft 7 and held by the protruding and recessed parts 25w formed from the outline of the strand 25, and the performance for recovering and transporting the substance in the body can be improved.

Note that, in the present embodiment, the strand 25 (corresponding to the "first strand" of the present invention) serving as the side wire is a strand consisting of seven wires, however the number of wires is not limited to seven, and may be a strand consisting of any number of two or more wires which enables a strand to be formed. However, from the viewpoint of the effect of the protruding and recessed parts 25W of the present embodiment, it is better to form the strand using the largest possible number of wires.

### (Second Embodiment)

Next, a second embodiment of the present invention will be described.

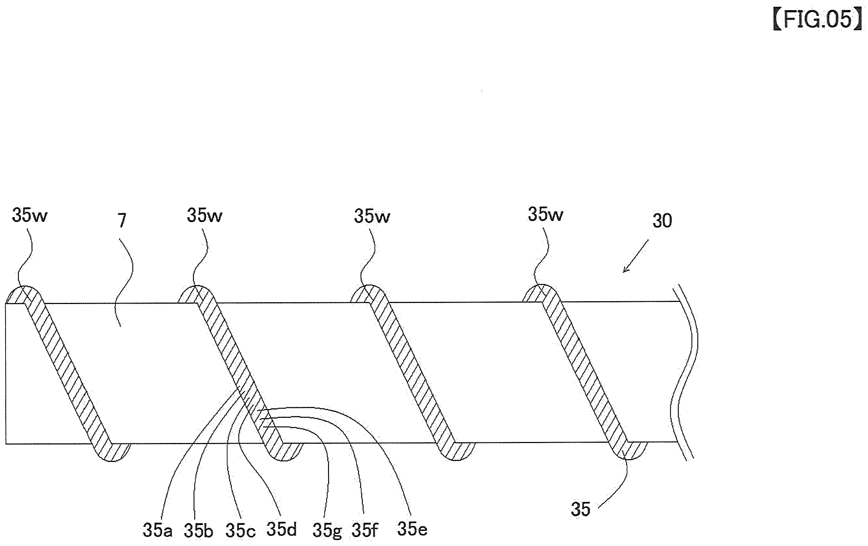

FIG. 5 is an enlarged view of a shaft for an in vivo recovery mechanism according to the second embodiment.

Hereinafter, the second embodiment of the present invention will be described, however, the in vivo recovery mechanism used in the shaft for an in vivo recovery mechanism of the present embodiment is identical to that of the shaft for an in vivo recovery mechanism 1 of the first embodiment, and the interior of the distal end of the in vivo recovery mechanism 1 is identical to FIG. 2, and therefore, the description thereof will be omitted, and further, those parts that are the same as the first embodiment will be referred to by the same reference numerals, and the description will be omitted.

The shaft 30 for an in vivo recovery mechanism of the present embodiment has the distal end connected to the cutter 2 through the bearing 3a connected to the interior of the distal end of the catheter main body 3b, the proximal end connected to the motor 8 through a bearing (not shown) connected to the interior of the proximal end of the catheter main body 3b, and is capable of rotating with the rotation of the motor 8.

As shown in FIG. 5, the shaft 30 for an in vivo recovery mechanism is constituted by the shaft 7 and a strand 35 (corresponding to a "first strand" of the present invention) serving as the side wire sparsely wound onto the outer circumference of the shaft 7, and the strand 35 is formed by twisting seven wires (corresponding to "first wires" of the present invention) 35a, 35b, 35c, 35d, 35e, 35f, and 35g.

Furthermore, the twisting direction of the strand 35 is a clockwise direction (hereinafter, referred to as "Z-twisting") toward the left direction of the drawing, and the winding direction of the strand 35 onto the shaft 7 is a Z-winding, such that the twisting direction of the strand 35 and the winding direction of the strand 35 onto the shaft 7 are the same direction.

Moreover, protruding and recessed parts 35w are formed from the outline of the strand 35, and the protruding and recessed parts 35w are formed around the entire outer circumferential surface of the strand 35. In addition, the protruding and recessed parts 35w have protruding parts and recessed parts, which are perpendicularly inclined in a direction parallel to the long axis direction of the shaft 7.

Although the material of the wires (corresponding to "first wires" of the present invention) 35a, 35b, 35c, 35d, 35e, 35f, and 35g constituting the strand 35 is not particularly limited if it is a biocompatible material such as stainless steel, tungsten, or Ni-Ti alloy, stainless steel is used in the present embodiment.

According to the shaft 30 for an in vivo recovery mechanism of the present embodiment, the twisting direction of the strand 35 serving as the side wire and the winding direction of the strand 35 onto the shaft 7 are the same direction, and therefore, the protruding and recessed parts 35w have protruding parts and recessed parts which are perpendicularly inclined in a direction parallel to the long axis of the shaft 7, and when rotating the shaft 30 for an in vivo recovery mechanism, the performance for recovering and transporting the substance in the body can be further improved.

Note that, in the present embodiment, the strand 35 (corresponding to a "first strand" of the present invention) serving as the side wire is a strand consisting of seven wires, however the number of wires is not limited to seven, and may be a strand consisting of any number of two or more wires which enables a strand to be formed. However, from the viewpoint of the effect of the protruding and recessed parts 35W of the present embodiment, it is better to form the strand using the largest possible number of wires.

### (Third Embodiment)

Next, a third embodiment of the present invention will be described.

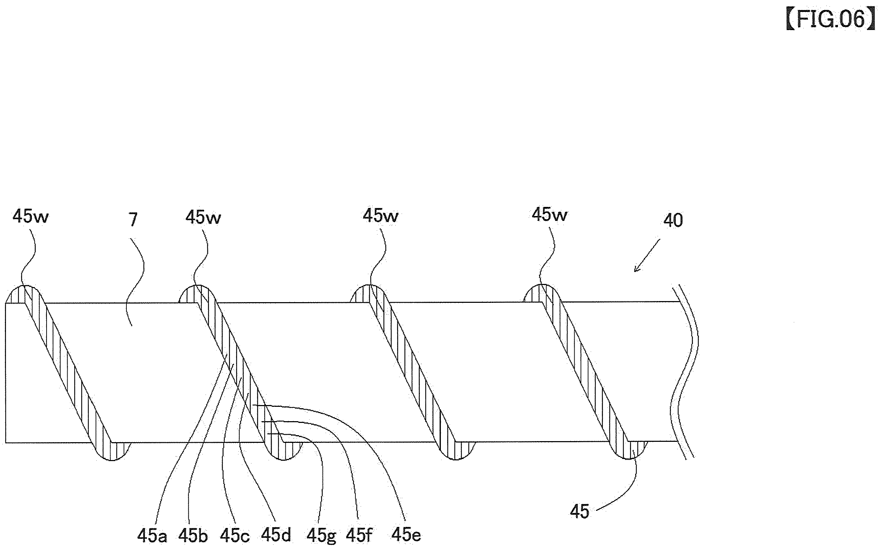

FIG. 6 is an enlarged view of a shaft for an in vivo recovery mechanism according to the third embodiment.

Hereinafter, the third embodiment of the present invention will be described, however, the in vivo recovery mechanism used in the shaft for an in vivo recovery mechanism of the present embodiment is identical to that of the shaft for an in vivo recovery mechanism 1 of the first embodiment, and the interior of the distal end of the in vivo recovery mechanism 1 is identical to FIG. 2, and therefore, the description thereof will be omitted, and further, those parts that are the same as the first embodiment will be referred to by the same reference numerals, and the description will be omitted.

The shaft 40 for an in vivo recovery mechanism of the present embodiment has the distal end connected to the cutter 2 through the bearing 3a connected to the interior of the distal end of the catheter main body 3b, the proximal end connected to the motor 8 through a bearing (not shown) connected to the interior of the proximal end of the catheter main body 3b, and is capable of rotating with the rotation of the motor 8.

As shown in FIG. 6, the shaft 40 for an in vivo recovery mechanism includes the shaft 7 and a strand 45 (corresponding to a "first strand" of the present invention) serving as the side wire sparsely wound onto the outer circumference of the shaft 7, and the strand 45 is formed by twisting seven wires (corresponding to "first wires" of the present invention) 45a, 45b, 45c, 45d, 45e, 45f, and 45g.

Furthermore, the twisting direction of the strand 45 is a Z-twisting, and the winding direction of the strand 45 onto the shaft 7 is a Z-winding, such that the twisting direction of the strand 45 and the winding direction of the strand 45 onto the shaft 7 are the same direction.

Moreover, protruding and recessed parts 45w are formed from the outline of the strand 45, and the protruding and recessed parts 45w are formed around the entire outer circumferential surface of the strand 45. In addition, the protruding and recessed parts 45w have protruding parts and recessed parts, which extend along a direction substantially perpendicular to the long axis direction of the shaft 7, in a direction parallel to the long axis direction.

Furthermore, although the material of the wires (corresponding to "first wires" of the present invention) 45a, 45b, 45c, 45d, 45e, 45f, and 45g constituting the strand 45 is not particularly limited if it is a biocompatible material such as stainless steel, tungsten, or Ni-Ti alloy, stainless steel is used in the present embodiment.

According to the shaft 40 for an in vivo recovery mechanism of the present embodiment, the protruding and recessed parts 45w have protruding parts and recessed parts, which extend along a direction substantially perpendicular to a long axis direction of the shaft 7, in a direction parallel to the long axis direction, and therefore, when the shaft 40 for an in vivo recovery mechanism is rotated, the performance for recovering and transporting the substance in the body can be further improved.

Note that, in the present embodiment, the strand 45 (corresponding to a "first strand" of the present invention) serving as the side wire is a strand consisting of seven wires, however the number of wires is not limited to seven, and may be a strand consisting of any number of two or more wires which enables a strand to be formed. However, from the viewpoint of the effect of the protruding and recessed parts 45W of the present embodiment, it is better to form the strand using the largest possible number of wires.

Furthermore, in the present embodiment, although the protruding and recessed parts 45w are formed from the outline of the strand 45 serving as the side wire, by application to the shaft 10 for an in vivo recovery mechanism of the first embodiment, protruding parts and recessed parts may be formed on the outer circumferential surface of the side wire 5 so as to extend along a direction substantially perpendicular to the long axis direction of the shaft 7, in a direction parallel to the long axis direction.

### (Fourth Embodiment)

Next, a fourth embodiment of the present invention will be described.

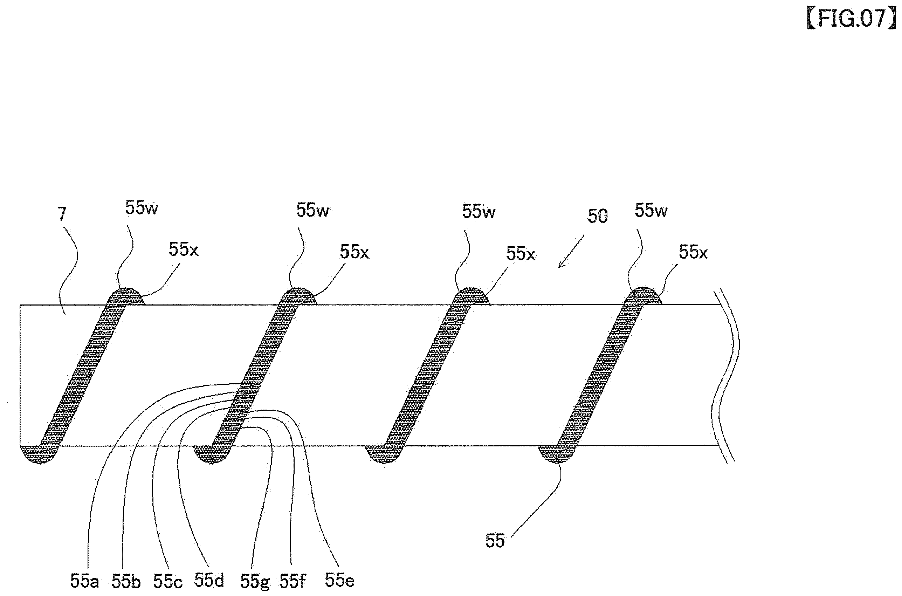

FIG. 7 is an enlarged view of a shaft for an in vivo recovery mechanism according to the fourth embodiment.

Hereinafter, the fourth embodiment of the present invention will be described, however, the in vivo recovery mechanism used in the shaft for an in vivo recovery mechanism of the present embodiment is identical to that of the shaft for an in vivo recovery mechanism 1 of the first embodiment, and the interior of the distal end of the in vivo recovery mechanism 1 is identical to FIG. 2, and therefore, the description thereof will be omitted, and further, those parts that are the same as the first embodiment will be referred to by the same reference numerals, and the description will be omitted.

The shaft 50 for an in vivo recovery mechanism of the present embodiment has the distal end connected to the cutter 2 through the bearing 3a connected to the interior of the distal end of the catheter main body 3b, and the proximal end connected to the motor 8 through a bearing (not shown) connected to the interior of the proximal end of the catheter main body 3b, and is capable of rotating with the rotation of the motor 8.

As shown in FIG. 7, the shaft 50 for an in vivo recovery mechanism is constituted by the shaft 7 and a strand 55 (corresponding to a "first strand" of the present invention) serving as the side wire sparsely wound onto the outer circumference of the shaft 7, and the strand 55 is formed by twisting seven wires (corresponding to "first wires" of the present invention) 55a, 55b, 55c, 55d, 55e, 55f, and 55g.

The twisting direction of the strand 55 is a Z-twisting, and the winding direction of the strand 55 onto the shaft 7 is a counterclockwise winding (hereinafter, referred to as "S-winding") toward the left direction of the drawing, such that the twisting direction of the strand 55 and the winding direction of the strand 55 onto the shaft 7 are opposite directions.

Moreover, protruding and recessed parts 55w are formed from the outline of the strand 55, and fine protruding and recessed parts 55x are formed on the outer circumferential surface of the wires (corresponding to the "first wires" of the present invention) 55a, 55b, 55c, 55d, 55e, 55f, and 55g constituting the strand 55.

The area in which the protruding and recessed parts 55x are formed is indicated by a hatching pattern in FIG. 7. That is to say, the protruding and recessed parts 55x in the present embodiment are formed on the entire outer circumferential surface of the wires 55a, 55b, 55c, 55d, 55e, 55f, and 55g.

In the present embodiment, the protruding and recessed parts 55x are formed on the entire outer circumferential surface of the wires 55a, 55b, 55c, 55d, 55e, 55f, and 55g, however they may be formed on part of the outer circumferential surface of the wires, and further, they may be formed on the entire outer circumferential surface of some of the wires and not formed at all on the outer circumferential surface of the other wires.

However, when the protruding and recessed parts 55x are formed on the entire outer circumferential surface of the wires 55a, 55b, 55c, 55d, 55e, 55f, and 55g, it is possible to improve the retention performance of the substance in the body and improve the recovery and transport performance to a greater extent.

Furthermore, although the material of the wires (corresponding to "first wires" of the present invention) 55a, 55b, 55c, 55d, 55e, 55f, and 55g constituting the strand 55 is not particularly limited if it is a biocompatible material such as stainless steel, tungsten, or Ni-Ti alloy, stainless steel is used in the present embodiment.

According to the shaft 60 for an in vivo recovery mechanism of the present embodiment, the protruding and recessed parts 55w are formed from the outline of the strand 55, and the protruding and recessed parts 55x are formed on the outer circumferential surface of the wires 55a, 55b, 55c, 55d, 55e, 55f, and 55g constituting the strand 55, and therefore, the performance for recovering and transporting the substance in the body can be further improved.

Note that, in the present embodiment, the strand 55 (corresponding to a "first strand" of the present invention) serving as the side wire is a strand consisting of seven wires, however the number of wires is not limited to seven, and may be a strand consisting of any number of two or more wires which enables a strand to be formed. However, from the viewpoint of the effect of the protruding and recessed parts 55W of the present embodiment, it is better to form the strand using the largest possible number of wires.

### (First Embodiment)

Next, a first embodiment of the present invention will be described.

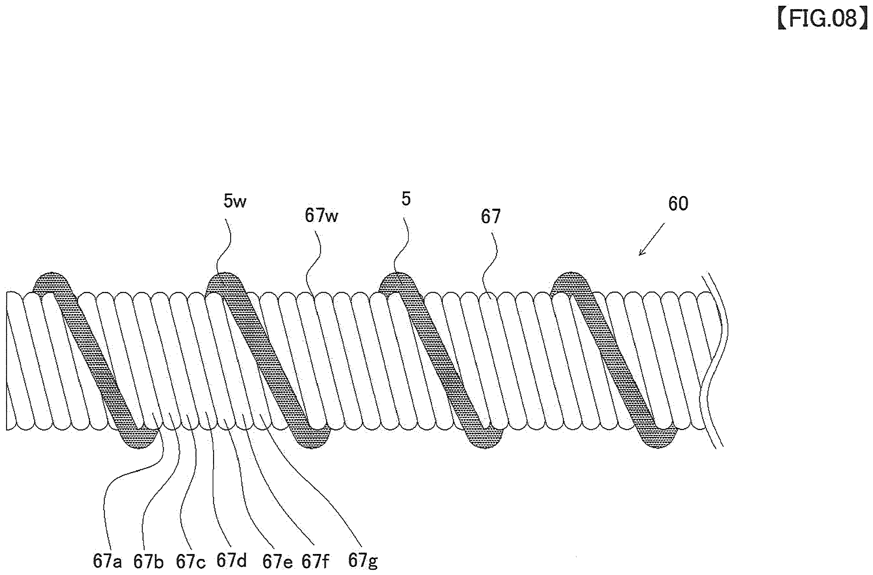

FIG. 8 is an enlarged view of a shaft for an in vivo recovery mechanism according to the first embodiment.

Hereinafter, the first embodiment of the present invention will be described, however, the in vivo recovery mechanism used in the shaft for an in vivo recovery mechanism of the present embodiment is identical to that of the shaft for an in vivo recovery mechanism 1 of the first embodiment, and the interior of the distal end of the in vivo recovery mechanism 1 is identical to FIG. 2, and therefore, the description thereof will be omitted, and further, those parts that are the same as the first embodiment will be referred to by the same reference numerals, and the description will be omitted.

The shaft 60 for an in vivo recovery mechanism of the present embodiment has the distal end connected to the cutter 2 through the bearing 3a connected to the interior of the distal end of the catheter main body 3b, and the proximal end connected to the motor 8 through a bearing (not shown) connected to the interior of the proximal end of the catheter main body 3b, and is capable of rotating with the rotation of the motor 8.

As shown in FIG. 8, the shaft 60 for an in vivo recovery mechanism includes a strand 67, which is a shaft formed by twisting seven wires (corresponding to "second wires" of the present invention) 67a, 67b, 67c, 67d, 67e, 67f, and 67g, and the side wire 5 sparsely wound onto the outer circumference of the strand 67.

Furthermore, the twisting direction of the strand 67 is a Z-twisting, and the winding direction of the side wire 5 onto the shaft 7 is a Z-winding, such that the twisting direction of the strand 67 and the winding direction of the side wire 5 onto the shaft 67 are the same direction.

Moreover, protruding and recessed parts 67w are formed from the outline of the strand 67.

Although the material of the wires (corresponding to "second wires" of the present invention) 67a, 67b, 67c, 67d, 67e, 67f, and 67g constituting the strand 67 is not particularly limited if it is a biocompatible material such as stainless steel, tungsten, or Ni-Ti alloy, stainless steel is used in the present embodiment.

According to the shaft 60 for an in vivo recovery mechanism of the present embodiment, the shaft is constituted by a strand 67 (corresponding to a "second strand" of the present invention) formed by twisting a plurality of wires 67a, 67b, 67c, 67d, 67e, 67f, and 67g, and therefore, the shaft 60 for an in vivo recovery mechanism is made softer, and further, when rotating the shaft 60 for an in vivo recovery mechanism, the substance in the body D is caught between the sparsely wound side wire 5 on the strand 67 and held by the protruding and recessed parts 67w formed from the strand 67 and the protruding and recessed parts 5w formed on the side wire 5, and the performance for recovering and transporting the substance in the body can be further improved.

Note that, in the present embodiment, the strand 67 (corresponding to a "second strand" of the present invention) serving as the shaft is a strand consisting of seven wires, however the number of wires is not limited to seven, and may be a strand consisting of any number of two or more wires which enables a strand to be formed. However, from the viewpoint of the effect of the protruding and recessed parts 67W of the present embodiment, it is better to form the strand using the largest possible number of wires.

Furthermore, the present embodiment was described by applying the side wire 5 of the first embodiment to the side wire, but the strand 25 of the first embodiment, the strand 35 of the second embodiment, the strand 45 of the third embodiment, and the strand 55 of the fourth embodiment may be applied instead of the side wire 5 sparsely wound onto the outer circumference of the strand 67 of the present embodiment, and in those cases, the effects of each alternative side wire described in the respective embodiments are provided in addition to the effects of the strand 67.

### (First Embodiment)

Next, a first embodiment of the present invention will be described.

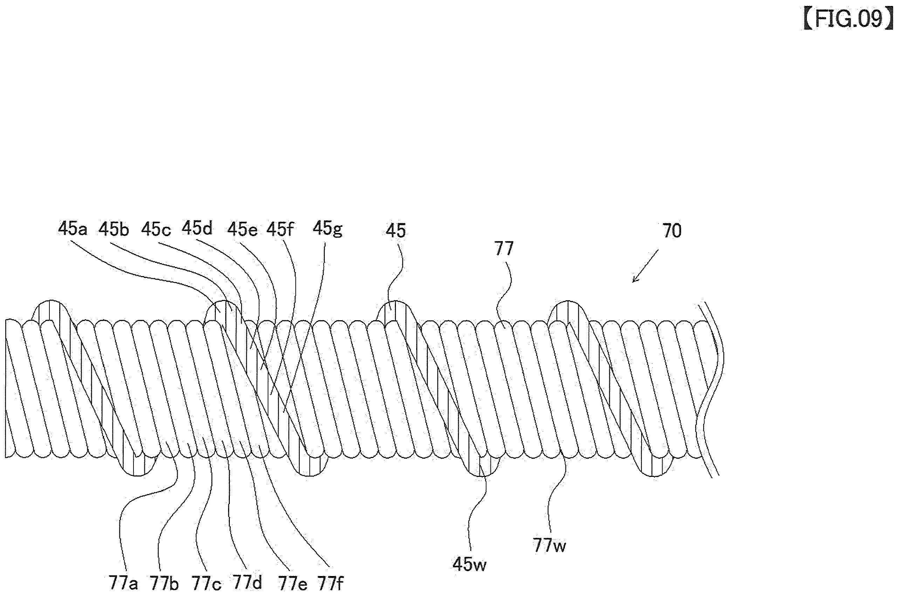

FIG. 9 is an enlarged view of a shaft for an in vivo recovery mechanism according to the first embodiment.

Hereinafter, the first embodiment of the present invention will be described, however, the in vivo recovery mechanism used in the shaft for an in vivo recovery mechanism of the present embodiment is identical to that of the shaft for an in vivo recovery mechanism 1 of the first embodiment, and the interior of the distal end of the in vivo recovery mechanism 1 is identical to FIG. 2, and therefore, the description thereof will be omitted, and further, those parts that are the same as the first embodiment will be referred to by the same reference numerals, and the description will be omitted.

The shaft 70 for an in vivo recovery mechanism of the present embodiment has the distal end connected to the cutter 2 through the bearing 3a connected to the interior of the distal end of the catheter main body 3b, the proximal end connected to the motor 8 through a bearing (not shown) connected to the interior of the proximal end of the catheter main body 3b, and is capable of rotating with the rotation of the motor 8.

As shown in FIG. 9, the shaft 70 for an in vivo recovery mechanism includes a hollow strand 77, which is a hollow shaft formed by twisting six wires (corresponding to "third wires" of the present invention) 77a, 77b, 77c, 77d, 77e, and 77f, and the strand 45 sparsely wound onto the outer circumference of the hollow strand 77.

Furthermore, the twisting direction of the hollow strand 77 is a Z-twisting, and the winding direction of the strand 45 onto the hollow strand 77 is a Z-winding, such that the twisting direction of the hollow strand 77 and the winding direction of the strand 45 onto the hollow strand 77 are the same direction.

Moreover, protruding and recessed parts 77w are formed from the outline of the hollow strand 77.

In addition, although the material of the wires (corresponding to "third wires" of the present invention) 77a, 77b, 77c, 77d, 77e, and 77f, constituting the hollow strand 77 is not particularly limited if it is a biocompatible material such as stainless steel, tungsten, or Ni-Ti alloy, stainless steel is used in the present embodiment.

According to the shaft 70 for an in vivo recovery mechanism of the present embodiment, the shaft is constituted by the hollow strand 77 formed by twisting the plurality of wires (corresponding to "third wires" of the present invention) 77a, 77b, 77c, 77d, 77e, and 77f, and therefore, the shaft 70 for an in vivo recovery mechanism is made softer, and further, when rotating the shaft 70 for an in vivo recovery mechanism, the substance in the body D is caught between the sparsely wound strand 45 on the hollow strand 77 and held by the protruding and recessed parts 77w formed from the hollow strand 77 and the protruding and recessed parts 45w formed on the side wire, and the performance for recovering and transporting the substance in the body can be further improved.

Furthermore, by inserting a guide wire or the like into a void inside the hollow strand 77 and causing the hollow strand 77 to be positioned along the guide wire, the shaft 70 for an in vivo recovery mechanism is capable of reaching the periphery of a body lumen such as a blood vessel.

Note that, in the present embodiment, the hollow strand 77 serving as the shaft is a strand consisting of seven wires, however the number of wires is not limited to seven, and may be a strand consisting of any number of two or more wires which enables a strand to be formed. However, from the viewpoint of the effect of the protruding and recessed parts 77W of the present embodiment, it is better to form the strand using the largest possible number of wires.

Furthermore, the present embodiment was described by applying the strand 45 of the third embodiment to the side wire, but the side wire 5 of the first embodiment, the strand 25 of the first embodiment, the strand 35 of the second embodiment, and the strand 55 of the fourth embodiment may be applied instead of the strand 45 sparsely wound onto the outer circumference of the strand 77 of the present embodiment, and in those cases, the effects of each alternative side wire described in the respective embodiments are provided in addition to the effects of the strand 77.

### DESCRIPTION OF REFERENCE NUMERALS

1 In vivo recovery mechanism
2 Cutter
3 Catheter
4 Cutter assembly
5 Side wire
5w, 25w, 35w, 45w, 55w, 55x, 67w, 77w Protruding and recessed parts
6 Grip part
7 Shaft
8 Motor
10, 20, 30, 40, 50, 60, 70 Shaft for an in vivo recovery mechanism
25, 35, 45, 55 Strand
77 Hollow strand
D Substance in the body
G Gap

## Claims

1. A shaft for an in vivo recovery mechanism for removing a substance from a body lumen (20,30,40,50,60,70) comprising:
a shaft (67,77); and
a side wire (25,35,45,55) sparsely wound onto an outer circumference of the shaft (67,77) and including protruding and recessed parts (25w,35w,45w,55w) on an outer circumferential surface of the side wire (25,35,45,55),
wherein the side wire (25,35,45,55) is constituted by a first strand (25,35,45,55) formed by twisting a plurality of first wires (25a,...,25e,35b,...35e,45a,...45e,55a,...55e), and
wherein the protruding and recessed parts (25w,35w,45w,55w) of the side wire (25,35,45,55) are formed from an outline of the first strand (25,35,45,55),
**characterised in that**
the shaft (67,77) is constituted by a second strand formed by twisting a plurality of wires (67a,67b,67c,67d,67e,67f,67g) or **in that** the shaft (67,77) is constituted by a hollow strand formed by twisting a plurality of third wires (77a,77b,77c,77d,77e,77f).

2. The shaft for an in vivo recovery mechanism for removing a substance from a body lumen (30,40,70) according to claim 1, wherein
a twisting direction of the first strand (35,45) is a same direction as a winding direction of the side wire (35,45) onto the shaft (7,77).

3. The shaft for an in vivo recovery mechanism for removing a substance from a body lumen (50) according to claim 1 or claim 2, wherein
protruding and recessed parts (55w) are formed on an outer circumferential surface of the wires constituting the first strand (55).

4. The shaft for an in vivo recovery mechanism for removing a substance from a body lumen (40,70) according to any one of claims 1 to 3, wherein
the protruding and recessed parts (45w) include protruding parts and recessed parts, which extend along a direction substantially perpendicular to a long axis direction of the shaft (7,77), in a direction parallel to the long axis direction.

## Patentansprüche

1. Schaft für einen In-vivo-Entnahmemechanismus zum Entfernen einer Substanz aus einem Körperlumen (20, 30, 40, 50, 60, 70), aufweisend:
einen Schaft (67, 77), und
einen seitlichen Draht (25, 35, 45, 55), der spärlich auf einen Außenumfang des Schafts (67, 77) gewickelt ist und vorstehende und vertiefte Teile (25w, 35w, 45w, 55w) auf einer Außenumfangsfläche des seitlichen Drahtes (25, 35, 45, 55) aufweist, wobei
der seitliche Draht (25, 35, 45, 55) durch eine erste Litze (25, 35, 45, 55) gebildet ist, die durch Verdrillen einer Vielzahl von ersten Drähten (25a, ..., 25e, 35b, ..., 35e, 45a, ..., 45e, 55a, ..., 55e) ausgebildet ist, und wobei
die vorstehenden und vertieften Teile (25w, 35w, 45w, 55w) des seitlichen Drahtes (25, 35, 45, 55) aus einem Umriss der ersten Litze (25, 35, 45, 55) gebildet sind, **dadurch gekennzeichnet, dass**
der Schaft (67, 77) durch eine zweite Litze gebildet ist, die durch Verdrillen einer Vielzahl von Drähten (67a, 67b, 67c, 67d, 67e, 67f, 67g) ausgebildet ist, oder dass der Schaft (67, 77) durch eine hohle Litze gebildet ist, die durch Verdrillen einer Vielzahl von Drähten (77a, 77b, 77c, 77d, 77e, 77f) ausgebildet ist.

2. Schaft für einen In-vivo-Entnahmemechanismus zum Entfernen einer Substanz aus einem Körperlumen (30, 40, 70) nach Anspruch 1, wobei
die Verdrillungsrichtung der ersten Litze (35, 45) die gleiche Richtung wie die Wickelrichtung des seitlichen Drahtes (35, 45) auf den Schaft (7, 77) ist.

3. Schaft für einen In-vivo-Entnahmemechanismus zum Entfernen einer Substanz aus einem Körperlumen (50) nach Anspruch 1 oder Anspruch 2, wobei
vorstehende und vertiefte Teile (55w) an einer Außenumfangsfläche der Drähte, die die erste Litze (55) bilden, ausgebildet sind.

4. Schaft für einen In-vivo-Entnahmemechanismus zum Entfernen einer Substanz aus einem Körperlumen (40, 70) nach einem der Ansprüche 1 bis 3, wobei
die vorstehenden und vertieften Teile (45w) vorstehende Teile und vertiefte Teile in einer Richtung parallel zu der Längsachsenrichtung umfassen, die sich entlang einer Richtung im Wesentlichen senkrecht zu einer Längsachsenrichtung des Schafts (7, 77) erstrecken.

## Revendications

1. Arbre pour un mécanisme de récupération in vivo pour éliminer une substance d'une lumière corporelle (20, 30, 40, 50, 60, 70), comprenant :
un arbre (67, 77) ; et
un fil latéral (25, 35, 45, 55) enroulé de manière clairsemée sur une circonférence externe de l'arbre (67, 77) et incluant des parties saillantes et en creux (25w, 35w, 45w, 55w) sur une surface circonférentielle externe du fil latéral (25, 35, 45, 55),
dans lequel le fil latéral (25, 35, 45, 55) est constitué d'un premier brin (25, 35, 45, 55) formé par torsion d'une pluralité de premiers fils (25a, ..., 25e, 35b, ..., 35e, 45a, ..., 45e, 55a, ..., 55e), et
dans lequel les parties saillantes et en creux (25w, 35w, 45w, 55w) du fil latéral (25, 35, 45, 55) sont formées à partir d'un contour du premier brin (25, 35, 45, 55),
**caractérisé en ce que** l'arbre (67, 77) est constitué d'un second brin formé par torsion d'une pluralité de fils (67a, 67b, 67c, 67d, 67e, 67f, 67g) ou **en ce que** l'arbre (67, 77) est constitué d'un brin creux formé par torsion d'une pluralité de fils (77a, 77b, 77c, 77d, 77e, 77f).

2. Arbre pour un mécanisme de récupération in vivo pour éliminer une substance d'une lumière corporelle (30, 40, 70) selon la revendication 1, dans lequel
un sens de torsion du premier brin (35, 45) est un même sens qu'un sens d'enroulement du fil latéral (35, 45) sur l'arbre (7, 77).

3. Arbre pour un mécanisme de récupération in vivo pour éliminer une substance d'une lumière corporelle (50) selon la revendication 1 ou la revendication 2, dans lequel
des parties saillantes et en creux (55w) sont formées sur une surface circonférentielle externe des fils constituant le premier brin (55).

4. Arbre pour un mécanisme de récupération in vivo pour éliminer une substance d'une lumière corporelle (40, 70) selon l'une quelconque des revendications 1 à 3, dans lequel
les parties saillantes et en creux (45w) incluent des parties saillantes et des parties en creux, qui s'étendent le long d'une direction sensiblement perpendiculaire à une direction d'axe long de l'arbre (7, 77), dans une direction parallèle à la direction d'axe long.
